# EUROPEAN PATENT SPECIFICATION

(11) **EP 2 379 056 B1**
(45) Date of publication and mention of the grant of the patent: **13.04.2016**
(21) Application number: 09767955.9
(22) Date of filing: 27.11.2009
(51) Int. Cl.: A61K 9/20

(54) **SOLID PHARMACEUTICAL COMPOSITION COMPRISING AT LEAST ONE STABILIZING AGENT**
FESTE PHARMAZEUTISCHE ZUSAMMENSETZUNG MIT MINDESTENS EINEM STABILISIERENDEN MITTEL
COMPOSITION PHARMACEUTIQUE SOLIDE INCLUANT AU MOINS UN AGENT STABILISANT

(30) Priority: 28.11.2008 SI 200800293
(43) Date of publication of application: 26.10.2011
(73) Proprietor: KRKA, d.d., Novo mesto, 8000 Novo mesto (SI)
(72) Inventor: SAGRAWAT, Hemant, Neemuch City 458 441 (IN); TENDULKAR, Anjali R., Thane (W) 400 605 (IN); KRISHNAN, Anandi, Vashi Navi Mumbai 400 703 (IN); KROSELJ, Vesna, 8310 Sentjernej (SI); GERMAN, Tamara, 8000 Novo mesto (SI); ZORKO, Karmen, 8250 Brezice (SI); KOLENC, Ivanka, 8000 Novo mesto (SI); TROST, Sabina, 1330 Kocevje (SI)
(74) Representative: Andrae | Westendorp Patentanwälte Partnerschaft
(86) International application number: PCT/EP2009/008467
(87) International publication number: WO 2010/060635

(56) References cited:
- EP-A1- 1 219 291
- WO-A2-03/028703
- WO-A2-2005/065047
- WO-A2-2006/011159
- WO-A2-2007/052289
- WO-A2-2007/149801
- KR-A- 20080 006 405

## Description

### Field of the Invention

The present invention is directed to a solid stable pharmaceutical composition comprising desloratadine and at least one stabilizing agent selected from the group consisting of alkali metal hydroxides, alkali earth metal hydroxides, and aluminium hydroxide,
said solid pharmaceutical composition being prepared by a process for the preparation of a solid pharmaceutical composition, wherein the process comprises or is wet granulation.

The invention further relates to a process of making said solid stable pharmaceutical composition comprising desloratadine and at least one stabilizing agent selected from the group consisting of alkali metal hydroxides, alkali earth metal hydroxides, and aluminium hydroxide.

### Background of the Invention

Histamine plays a major role in the pathogenesis of allergic rhinitis and urticaria, primarily via the H₁-histamine receptor in target tissues. H₁-antihistamines have therefore been the main focus of drug development for the treatment of these disorders.

Histamine H₁ receptor antagonists represent the most widely used class of drugs for the treatment of allergic disorders, especially rhinitis and urticaria. Compounds with this mechanism of action block the effects of histamine at H₁ histamine receptors on postcapillary venule smooth muscles, resulting in decreased capillary permeability, reduced mucus production, relaxation of smooth muscle, and reduced vasodilatation. They also prevent histamine from acting on H₁ receptors on type-C nociceptive nerve fibers, thus reducing allergy-related symptoms of sneezing and itching.

First generation H₁-antihistamines such as chlorphenamine and diphenhydramine provide symptomatic relief of allergic rhinitis and urticaria but are associated with undesirable CNS effects such as sedation and impaired psychomotor activity at therapeutic dosages, and adverse anticholinergic effects due to their high blood-brain barrier penetration. The H₁-receptor-selective second generation agents such as loratadine, cetirizine, fexofenadine, astemizole and terfenadine exhibit fewer sedative and anticholinergic effects, but use of the latter two agents has been associated with adverse cardiovascular effects so both agents were withdrawn. While second generation antihistamines are highly effective in treating most symptoms of allergic rhinitis, they are generally not effective against nasal congestion. The third generation H₁-antihistamine desloratadine is the orally active major metabolite of the nonsedating antihistamine loratadine.

Desloratadine is a non-sedating long acting histamine antagonist with selective peripheral H₁-receptor antagonist activity indicated for the relief of symptoms associated with allergic rhinitis and urticaria.

Desloratadine, commonly referred to as descarboethoxyloratadine, is chemically known as 8-chloro-6,11-dihydro-11-(4-piperidinylidene)-5H-benzo[5,6]cyclo-hepta[1,2-*b*]pyridine and is disclosed in EP 0 152 897. Desloratadine is known to be a sensitive molecule that is found to discolour and decompose due to moisture and/or humidity conditions and elevated temperature. According to EP 1073438 the colour instability in the active ingredient was attributed to a very minute amount of a degradation product, the *N*-formyl desloratadine, which is formed due to the presence of a wide variety of excipients commonly used in oral formulations-especially tablet formulation. These excipients found unsuitable include acidic excipients as well as reactive excipients.

Furthermore, EP 1073438 teaches stabilization of desloratadine by addition of a pharmaceutically acceptable carrier medium comprising a desloratadine protective amount of a pharmaceutically acceptable basic salt such as carbonate, phosphate, silicate or sulphate of calcium, magnesium or aluminium or mixtures thereof while avoiding the use of acidic excipients.

EP 969836 addresses the issue of the instability and degradation of desloratadine in the presence of lactose or other reactive excipients such as other mono- or disaccharide excipients. It teaches that the amount of reactive excipients or lactose as appropriate, present, in the dosage form or pharmaceutical composition of desloratadine according to said patent, is insufficient to cause incompatibility between desloratadine and the particular excipients, such as lactose. It further discloses that pharmaceutical compositions of desloratadine containing lactose, or other reactive excipients, that are exposed to moisture and humidity degrade more rapidly and suggests that substantial contact with humidity and/or moisture during manufacture, handling, packaging, storage, shipment and use of the formulation should be avoided.

Accordingly, EP 969836 discloses a lactose-free pharmaceutical composition comprising a therapeutically effective amount of desloratadine or a pharmaceutically acceptable salt thereof, which has been granulated with a pharmaceutically acceptable inert carrier.

WO 2005/065047 discloses a pharmaceutical composition comprising desloratadine and a stabilizer selected from an antioxidant, a pharmaceutically acceptable organic compound that provides an alkaline pH, an alkali metal salt, and mixtures thereof, and pharmaceutically acceptable excipients.
Example 4 of WO 2005/065047 A2 discloses coated tablets comprising desloratadine and disodium hydrogen phosphate.

The pharmaceutical composition of WO 2006/008512 comprises desloratadine and a carrier comprising at least one polyol while avoiding the use of stearic acid and derivatives thereof.

According to KR 20080006405 (A), a pharmaceutical composition is provided to simply prepare a tablet having the mechanical strength of 4-5 kp, which is rapidly disintegrated orally to promote the absorption of an active ingredient in vivo. This quick-dissolved pharmaceutical composition comprises 1-30 wt.% of loratadine or desloratadine.

According to a package leaflet provided by the manufacturer SP Labo N.V., Aerius^{®} 5 mg film-coated tablets comprise 5 mg desloratadine and other ingredients, such as calcium hydrogen phosphate dihydrate.

WO 2006/011159 A2 relates to stable pharmaceutical composition of rabeprazole sodium in a form of enteric coated pellets, either alone or in combination with one or more prokinetic agent, preferably mosapride in a form of sustained release tablets, in a single unit dosage form, which provides enhanced bioavailability of rabeprazole.

WO 2007/140987 discloses a pharmaceutical composition of desloratadine that can be prepared without addition of a basic salt while still using acidic components.

According to the state of the art mentioned above there remains a need for a pharmaceutical composition of desloratadine that is stable under pharmaceutical storage conditions and can be easily prepared. In particular, there is a need in the art for a pharmaceutical composition which has sufficient or good storage stability, preferably an improved storage stability under pharmaceutical storage conditions when compared to at least one commercially available solid pharmaceutical composition comprising desloratadine.

### Brief description of the drawings

Fig. 1a represents an XRD diffractogram of the solid pharmaceutical composition obtained according to Example 3.
Fig. 1b represents an XRD diffractogram of the solid pharmaceutical composition without the active ingredient desloratadine obtained according to Example 3.
Fig. 2a represents an XRD diffractogram of the solid pharmaceutical composition obtained according to Example 3 measured after 15 days storage in open containers at 40 °C/75 % RH (relative humidity).
Fig. 2b represents an XRD diffractogram of the solid pharmaceutical composition without the active ingredient desloratadine obtained according to Example 3 measured after 15 days storage at 40 °C/75 % RH.
   The XRD diffractograms were recorded on a Phillips PW3040/60 X'Pert PRO diffractometer using CuKα radiation (1,541874 A).

### Detailed description

According to one aspect, the present inventors provide a solid pharmaceutical composition as defined in independent claim 1.

According to a further aspect, the present inventors provide a process for the preparation of a solid pharmaceutical composition as defined in claim 7.

The present invention is directed to a solid, in particular a solid stable, pharmaceutical composition comprising desloratadine and at least one stabilizing agent selected from the group consisting of alkali metal hydroxides and alkali earth metal hydroxides, and aluminium hydroxide.

The term "solid pharmaceutical composition" encompasses, but is not restricted to, pharmaceutical compositions being in the solid state at least at room temperature (20°C).

The invention further relates to a process of making said solid, in particular said solid stable, pharmaceutical composition comprising desloratadine and at least one stabilizing agent selected from the group consisting of alkali metal hydroxides, alkali earth metal hydroxides, and aluminium hydroxide.

Active ingredient desloratadine in the context of the present invention can be present, for example in the pharmaceutical composition and/or as starting material for preparing the pharmaceutical composition, in the free base form or in the form of a pharmaceutically acceptable salt, enantiomer, derivative, hydrate or solvate thereof. It may further be present in a crystalline or non-crystalline form such as a polymorphic, pseudopolymorphic or amorphous form.

Alkali metal hydroxides, alkali earth metal hydroxides, and aluminiumhydroxide, in particular NaOH, KOH, Ca(OH)₂, Mg(OH)₂, Al(OH)₃,can be used in any pharmaceutically acceptable form, for example in form of hydrate(s) or solvate(s) thereof, in crystalline or non-crystalline form(s), such as amorphous form(s).

Furthermore, the term "alkali metal hydroxides" encompasses also mixed alkali metal hydroxides comprising more than one type of alkali metal ions and/or metal ions different from alkali metal ions, said metal ions different from alkali metal ions being preferably pharmaceutically acceptable metal ions and can be preferably present in the mixed hydroxide in a ratio of (molar amount of metal ions different from alkali metal ions) to (molar amount of alkali metal ions) of not more than 1:1, preferably of not more than 1:2.

The term "earth alkali metal hydroxides" encompasses also mixed earth alkali metal hydroxides comprising more than one type of earth alkali metal ions and/or metal ions different from earth alkali metal ions, said metal ions different from earth alkali metal ions being preferably pharmaceutically acceptable metal ions and can be preferably present in the mixed hydroxide in a ratio of (molar amount of metal ions different from earth alkali metal ions) to (molar amount of earth alkali metal ions) of not more than 1:1, preferably not more than 1:2.

The term "aluminium hydroxide" encompasses also mixed aluminium metal hydroxides comprising metal ions different from aluminium ions, said metal ions different from aluminium ions being preferably pharmaceutically acceptable metal ions and can be preferably present in the mixed hydroxide in a ratio of (molar amount of metal ions different from aluminium ions) to (molar amount of aluminium ions) of not more than 1:1, preferably of not more than 1:2.

Furthermore, the terms "alkali metal hydroxides", "earth alkali metal hydroxides", and "aluminium hydroxide" also encompass alkali metal oxide-hydroxides, earth alkali metal oxide-hydroxides, and aluminium oxide-hydroxides, respectively.

Preferably "alkali metal hydroxides", "earth alkali metal hydroxides", and "aluminium hydroxide" can be free or essentially free of mixed hydroxides and oxide-hydroxides.

It is an object of the present invention to provide a further simplified and thus inexpensive pharmaceutical composition comprising desloratadine, which composition is stable even in the presence of reactive excipients and can advantageously be prepared on an industrial scale.

It has surprisingly been found that this object can be achieved by a solid pharmaceutical composition comprising desloratadine, at least one stabilizing agent selected from the group consisting of alkali metal hydroxides and alkali earth metal hydroxides and aluminium hydroxide, and optionally one or more pharmaceutically acceptable excipients.

In particular, alkali metal hydroxides can be selected from the group consisting of sodium hydroxide, potassium hydroxide, lithium hydroxide and mixtures thereof.

In particular, alkali earth metal hydroxides can be selected from the group consisting of calcium hydroxide, magnesium hydroxide and mixtures thereof.

The at least one stabilizing agent according to the present invention can be preferably selected from sodium hydroxide, potassium hydroxide, calcium hydroxide, magnesium hydroxide, and aluminium hydroxide, more preferably from sodium hydroxide and potassium hydroxide.

The or a stabilizing agent can be present, in particular in a solid pharmaceutical composition of the present invention, in the amount of 0.001 to 10.00 weight%, preferably 0.003 to 5.0 weight%, more preferably 0.005 to 2.5 weight%, calculated on the weight of the solid pharmaceutical composition.

In particular, in a solid pharmaceutical composition of the present invention one or more stabilizing agent(s) selected from the group consisting of alkali metal hydroxides, alkali earth metal hydroxides, and aluminium hydroxide, preferably selected from the group consisting of NaOH, KOH, Ca(OH)₂, Mg(OH)₂, and Al(OH)₃ can be each present in an amount of 0.001 to 10.00 weight%, preferably of 0.003 to 5.0 weight%, more preferably of 0.005 to 2.5 weight%, calculated on the weight of the solid pharmaceutical composition. Furthermore, in a solid pharmaceutical composition of the present invention, the total amount of stabilizing agent(s), selected from the group consisting of alkali metal hydroxides, alkali earth metal hydroxides, and aluminium hydroxide, preferably selected from the group consisting of NaOH, KOH, Ca(OH)₂, Mg(OH)₂, and Al(OH)₃ can be present in an amount of 0.001 to 10.00 weight%, preferably 0.003 to 5.0 weight%, more preferably 0.005 to 2.5 weight%, calculated on the weight of the solid pharmaceutical composition.

It has surprisingly also been found that the solid pharmaceutical composition comprising desloratadine and at least one stabilizing agent according to the present invention shows a high stability, in particular a high stability during storage and/or with respect to degradation of desloratadine, even if only a small amount of the above described stabilizing agent is used. The compositions according to the present invention are simple and can easily be produced by state of the art technological processes. Particularly advantageous pharmaceutical compositions can be obtained when producing the compositions of the present invention by carrying out a process as described in detail below.

Desloratadine can generally be prepared by any known process such as the processes described in EP 0 152 987, EP 0 535 152, EP 1 542 986, EP 1 347 965 and WO 2008/032136.

According to the present invention desloratadine can be present, partially or completely, in amorphous form and/or can be present, partially or completely, in different crystalline forms, in particular in a polymorphic form, such as polymorphic form I, polymorphic form II or mixtures thereof such as for example disclosed in EP 993455, EP 1507531, EP 1862462 and WO 2007/140987.

It is preferred that desloratadine is present in amorphous form. For example, amorphous desloratadine can be prepared according to the process described in WO 2005/084674 or during the technological process of the preparation of the solid pharmaceutical composition according to the present invention.

In particular, the amorphous form of desloratadine can be prepared during the process for the preparation of said solid pharmaceutical composition, said solid pharmaceutical composition being preferably obtainable by a process comprising: providing desloratadine completely or at least partially in a form different from the amorphous form of desloratadine, and converting the desloratadine present in a form different from the amorphous form of desloratadine completely or at least partially into desloratadine in amorphous form.

Preferable average particle size of active ingredient of the present invention, in particular the preferable average particle size of particles consisting of or comprising desloratadine, depends on the technological process used. For example, if active ingredient, in particular desloratadine, is dissolved and/or suspended in suitable solvent and sprayed onto the carrier, average particle size, in particular the average particle size of particles consisting of or comprising desloratadine, can be of the range of 1 µm to 500 µm, preferably 2 µm to 250 µm. In case of a wet granulation process, preferable average particle size of the active ingredient, in particular the preferable average particle size of particles consisting of or comprising desloratadine, can be 2 µm to 250 µm. In case of direct compression, preferable average particle size, in particular the preferable average particle size of particles consisting of or comprising desloratadine, can be less than 150 µm.

The term "average particle size" as used herein refers to the volume mean diameter of particles. The diameter and volume mean diameter can be determined by laser light scattering using e.g. a Malvern-Mastersizer Apparatus MS 2000. Particle sizes are determined by measuring the angular distribution of laser light scattered by a homogeneous suspension of particles. The particles to be subjected to the particle size measurement are first suspended in appropriate non-polar dispersant and then subjected to a size determination in a Malvern Mastersizer MS 2000 instrument. Usually, 100-800 mg of substance are dispersed in 5-10 ml of dispersant. In particular, 100 mg of substance can be dispersed in 10 ml of vegetable oil at a room temperature.

The amount of the active ingredient desloratadine present in the solid pharmaceutical composition according to the present invention can be 0.1-15 mg, preferably 1-10 mg and most preferably 2.5-5 mg of desloratadine per final dosage form, for example per tablet or capsule.

The solid pharmaceutical composition according to the present invention in addition to the active ingredient desloratadine and at least one stabilizing agent, further comprises optionally one or more pharmaceutically acceptable excipients. Suitable excipient(s) can be selected from the group consisting of, but are not limited to, a diluent, a binder, a disintegrant, a lubricant, a glidant, an antioxidant, a crystallization inhibitor, and optionally flavoring and/or sweetening agents, and mixtures thereof. Optionally, the solid pharmaceutical composition can be further coated.

The solid pharmaceutical composition according to the present invention can be present in the form of tablets, orodispersible tablets, pills, powders, lozenges, sachets, capsules, in particular soft and hard gelatine capsules, (filled with powders, granules or pellets, in particular filled with powder(s), granules or pellets comprising or consisting of a composition comprising desloratadine and one or more of the at least one stabilizing agent(s)), suppositories etc. Preferably, the dosage form is suitable for oral application.

The diluent can be selected from the group consisting of, but is not limited to, microcrystalline cellulose, powdered cellulose, composite materials combining crystalline cellulose with lactose (Cellactose), guar gum (Avicel CE15) or silicified cellulose (Prosolv), corn starch, maize starch, starch derivatives such as pregelatinized starch, calcium hydrogen phosphate in anhydrous and hydrated form, various types of sugars such as lactose (anhydrous and monohydrate), compressible sugar, fructose, dextrates, sugar alcohols such as mannitol, sorbitol, maltitol, xylitol, lactitol, isomalt or other sugars such as saccharose, raffinose, trehalose, fructose or mixture thereof, calcium carbonate, calcium lactate or mixture thereof, and mixtures of these compounds and mixtures. The diluent can be present in an amount of 10 to 99 weight%, preferably 25 to 90 weight% calculated on the weight of the solid pharmaceutical composition.

The binder can be selected from the group consisting of, but is not limited to, polyvinylpyrrolidone, microcrystalline cellulose, cellulose ether such as for example hydroxyethylcellulose, hydroxypropylcellulose, and hydroxypropylmethylcellulose of different grades (i.e. viscosity), corn starch, maize starch, pregelatinised starch, polymethacrylate, or mixtures thereof. The binder can be present in a range of 0.5 to 25 weight%, preferably 1 to 20 weight% calculated on the weight of the solid pharmaceutical composition.

The disintegrant can be selected from the group consisting of, but is not limited to, crospovidone, starch, starch derivatives such as pregelatinised starch and sodium starch glycollate, microcrystalline cellulose, carboxymethylcellulose sodium (CMC-Na) and/or calcium (CMC-Ca), cross-linked CMC-Na, polacrilin potassium, low-substituted hydroxypropylcellulose or mixtures thereof and can be present in an amount of 1 to 50 weight%, preferably 2 to 45 weight%, based on the weight of the solid pharmaceutical composition.

The lubricant can be selected from the group consisting of, but is not limited to, stearic acid, magnesium stearate, magnesium palmitate, magnesium oleate, magnesium lauryl sulfate, hydrogenated vegetable oil, hydrogenated castor oil, talc, sodium stearyl fumarate, glyceryl behanate, macrogols or mixtures thereof and can be present in a range of 0.1 to 10 weight%, preferably 0.5 to 5 weight% based on the weight of the solid pharmaceutical composition.

The glidant can be selected from the group consisting of, but is not limited to, colloidal silicon dioxide, talc, stearic acid, palmitic acid, polyethylene glycol, carnauba wax or mixtures thereof.

The crystallization inhibitor can be selected from the group consisting of, but is not limited to, cellulose derivatives such as hydroxypropylmethylcellulose (HPMC), ethyl cellulose, sodium or calcium carboxymethylcellulose, hydroxyethylcellulose, HPMC phthalate, polyvinylpyrrolidone and derivatives thereof, polyvinyl alcohol, polyethylene glycol, block copolymers of ethylene oxide or propylene oxide, xanthan gums, pectins, alginates, tragacanth and derivatives, gum arabic and derivatives, carrageenans, agar and derivatives, polysaccharides from microbiological sources, acacia, starch, arabinogalactanes, galactomannans, dextrans and the like can be present in a range of 0.1 to 15 weight%, preferably 1 to 10 weight% based on the weight of the solid pharmaceutical composition.

The sweetener can be selected from the group consisting of, but is not limited to, acesulfame K, sucralose, alitame, aspartame, saccharin sodium, dipotassium glycyrrhizinate, stevia and thaumatin and the like.

The flavouring agent can be selected from the group consisting of, but is not limited to, natural or synthetic materials, such as orange, spearmint, strawberry and cream flavour and the like.

The composition according to the present invention may further comprise one or more antioxidants selected from the group consisting of but not limited to alkyl gallates (e.g. dodecyl-, ethyl-, octyl-, propyl-gallate), butylated hydroxyanisole, butylated hydroxytoluene, tocopherols (e.g. alpha tocopherol), ascorbic acid palmitate, ascorbic acid, sodium ascorbate, potassium and sodium salts of sulphurous acid (e.g. bisulphites, metabisulphites, sulphites), flavonoides (rutin, quercetin, caffeic acid).

Optionally, cores/tablets can be coated with conventional materials used for film coating, i.e. as described in Pharmaceutical Coating Technology (G. Cole (ed.), 1995). Film coating formulations may usually contain the following components:
- polymer(s),
- plasticizer(s),
- colourant(s)/opacifier(s),
- vehicle(s).

In a film coating suspension additional quantities of flavours, surfactants, adhesion enhancers such as for example saccharides selected from but not limited to the group consisting of polydextrose, maltodextrin and lactose and waxes can be used. The majority of the polymers used in a film coating can be either cellulose derivatives, such as the cellulose ethers, or acrylic polymers and copolymers. Occasionally encountered can be high molecular weight polyethylene glycols, polyvinyl pyrrolidone, polyvinyl alcohol and waxy materials. Their function usually is to prevent bad mouth feel and/or taste and in some cases degradation, e.g. oxidation of the active ingredients and/or excipients used.

Typical cellulose ethers which may be applied as coatings are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The commonly used plasticizers can be categorized into three groups:
- polyols such as glycerol, propylene glycol, macrogols,
- organic esters such as phthalate esters, dibutyl sebacetate, citrate esters, triacetin,
- oils/glycerides such as castor oil, acetylated monoglycerides, fractionated coconut oil.

Colourants/opacifiers are classified into several groups:
- organic dyes and their lakes,
- inorganic colours,
- natural colours.

Different materials from each group can also be combined in defined ratios. Film coating suspensions can also be used as ready-to-make preparations which are available on the market such as for example Opadry®, Opadry® II, Opadry® AMB, Opadry® fx, Opadry® ns-g, Opadry® NS, Opadry® tm etc.

Film coating dispersion can be prepared by using different solvents such as water, alcohols, ketones, esters, chlorinated hydrocarbons, preferably water is used.

A composition of coating suspension (calculated on dry material) which comprises:
- 1-99%, preferably 1-95% by weight of polymer,
- 1-50%, preferably 1-40% by weight of plasticizer,
- 1-50%, preferably 1-40% by weight of adhesion enhancer,
- 0.1-20%, preferably 0.1-10% by weight of colourant/opacifier,
is particularly preferred.

Conventional equipment for applying a coating, such as conventional coating pans or a Wurster coating system can be used.
According to a particular embodiment of the invention, the solid pharmaceutical composition can be obtained by known technological procedures, e.g. by direct compression, dry, wet or spray granulation, using well known and readily available excipients. Pharmaceutical compositions showing a particularly good storage behavior can be obtained, when carrying out steps of the preparation process as described in detail below. In the preparation of the compositions of desloratadine, the active ingredient can usually be mixed with an excipient or mixture of excipients, or diluted with an excipient or mixture of excipients. When the excipient serves as a diluent, it may be solid or liquid material which acts as a vehicle or medium for desloratadine.

According to one embodiment of the present invention, the total weight of stabilizing agents selected from the group consisting of alkali metal hydroxides, alkali earth metal hydroxides, and aluminium hydroxide, preferably selected from the group consisting of NaOH, KOH, Ca(OH)₂, Mg(OH)₂, and Al(OH)₃, more preferably selected from the group consisting of NaOH and KOH, and the total weight of desloratadine present in and/or added during the preparation of the pharmaceutical composition may be present in a weight ratio of (total weight of said stabilizing agents) : (total weight of desloratadine) of from 0.001:1 to 3:1, preferably from 0.01:1 to 2:1, more preferably from 0.1:1 to 1:1, and/or desloratadine may be present in the solid pharmaceutical composition in an amount of 0.1 to 90 weight%, preferably in an amount of 2 to 20 weight%, more preferably in an amount of 3 to 8 weight%, each based on the weight of the solid pharmaceutical composition.

The process for preparing the pharmaceutical composition according to the invention is carried out as a granulation process and optionally direct compression process. The granulation process is or comprises wet granulation and optionally spray granulation and optionally dry granulation.

In a preferred embodiment (a), wet granulation process comprises:
a) granulating a mixture of excipients using a granulation liquid to obtain a granulate,
b) contacting, preferably spraying, the obtained granulate with a dispersion or a solution of a granulation liquid comprising desloratadine,
c) optional addition of further excipient(s) to the granulate, preferably to the granulate resulting from step b), to give a compression mixture,
d) compressing the compression mixture to a desired form, and
e) optionally, applying a coating.

In another preferred embodiment (b), wet granulation process comprises:
a) granulating a mixture of excipient(s), using a granulating liquid comprising desloratadine to obtain a granulate,
b) optional addition of further excipient(s) to the granulate to give a compression mixture,
c) compressing the compression mixture to a desired form, and
d) optionally, applying a coating.

In another preferred embodiment (c), the wet granulation process comprises:
a) mixing of desloratadine with at least one excipient,
b) granulating the mixture of desloratadine and excipient(s), using granulating liquid to obtain a granulate,
c) optional addition of further excipient(s) to the granulate to give a compression mixture,
d) compression of the obtained mixture to a desired form, and
e) optionally, applying a coating.

In another preferred embodiment (d), wet granulation process comprises:
a) granulating a mixture of excipient(s), using a granulation liquid comprising desloratadine to obtain a granulate,
b) contacting, preferably spraying, the obtained drug loaded granules with granulation liquid,
c) optional addition of further excipient(s) to the granulate to give a compression mixture,
d) compressing the compression mixture to the desired form, and
e) optionally, applying a coating.

In more preferred embodiment, wet granulation process comprises:
a) granulating a mixture of excipient(s), using a granulation liquid comprising desloratadine to obtain a granulate,
b) contacting, preferably spraying, the obtained drug loaded granules with granulation liquid comprising a stabilizing agent, preferably with a granulation liquid comprising at least one stabilizing agent selected from the group consisting of alkali metal hydroxides, alkali earth metal hydroxides, and aluminium hydroxide,
c) optional addition of further excipient(s) to the granulate to give a compression mixture,
d) compressing the compression mixture to a desired form, and
e) optionally, applying a coating.

In the most preferred embodiment, wet granulation process comprises:
a) granulating a mixture of excipient(s), using a granulation liquid comprising desloratadine to obtain a granulate,
b) contacting, preferably spraying, the obtained drug loaded granules with granulation liquid comprising stabilizing agent(s) selected from sodium hydroxide and potassium hydroxide,
c) optional addition of further excipient(s) to the granulate, preferably to the granulate resulting from step b), to give compression mixture,
d) compressing the compression mixture to a desired form, and
e) optionally, applying a coating.

A process for the preparation of a solid pharmaceutical composition can further comprise
- providing desloratadine completely or at least partially present in a form different from the amorphous form of desloratadine,
- preparing a granulation liquid from said desloratadine completely or at least partially present in a form different from the amorphous form of desloratadine.

Moreover, a process for the preparation of a solid pharmaceutical composition can further comprise
- obtaining a pharmaceutical composition comprising desloratadine completely or at least partially present in amorphous form, preferably obtaining a pharmaceutical composition having a weight ratio of desloratadine present in amorphous form to desloratadine present in a form different from amorphous form which is higher than the weight ratio of desloratadine present in amorphous form to desloratadine present in a form different from amorphous form present in the desloratadine provided for preparing the granulation liquid comprising desloratadine.

According to another aspect, the present invention provides a pharmaceutical composition obtainable according to any of the before-mentioned processes. Due to the specific process steps described above, pharmaceutical substances with a specific internal structure and surface properties are obtained, providing particularly advantageous properties to the pharmaceutical composition.

According to yet another aspect, a use of a pharmaceutical composition according to the present invention for the manufacture of a medicament for treating, preventing or alleviating allergic rhinitis and/or urticaria, in particular sneezing and itching associated with allergic rhinitis, and nasal congestion, is provided.

According to yet another aspect, a pharmaceutical composition according to the present invention for use in treatment, prevention or alleviation of allergic rhinitis and/or urticaria, in particular sneezing and itching associated with allergic rhinitis, and nasal congestion, is provided.

The terms "granulating liquid" and "granulation liquid" according to the present invention refer to water, alcohol, water/alcohol mixture, or an aqueous, alcoholic or aqueous/alcoholic solution, dispersion or suspension, which contains purified water or demineralised water or lower alcohols such as methanol, ethanol, isopropanol or mixtures thereof as diluents and the substance which is dispersed, suspended or dissolved in the diluent. The said substance can have known functions of excipients used in the solid pharmaceutical composition according to the invention such as binder, surfactant, stabilizing agent or crystallization inhibitor.

In the context of the present application, the terms "granulating liquid" and "granulation liquid" are used interchangeably.

The terms "granulating liquid comprising desloratadine" and "granulation liquid comprising desloratadine" according to the present invention refer to water, alcohol, water/alcohol mixture containing desloratadine, or an aqueous, alcoholic or aqueous/alcoholic solution, dispersion or solution, which contains purified water or demineralised water or lower alcohols such as methanol, ethanol, isopropanol or mixtures thereof as diluents, desloratadine and the substance which is dispersed, suspended or dissolved in the diluent. The said substance can have known functions of excipients used in the solid pharmaceutical composition according to the invention such as binder, surfactant, stabilizing agent or crystallization inhibitor.

In the context of the present application, the terms "granulating liquid comprising desloratadine" and "granulation liquid comprising desloratadine" are used interchangeably.

Granulation liquid comprising desloratadine can be prepared by mixing/dissolving/suspending/dispersing desloratadine and optionally other excipients in a diluent.

Granulation liquid comprising desloratadine can be prepared by dissolving desloratadine in acidic water solution. For example, HCl, or different organic acids such as citric acid and ascorbic acid can be used. Granulation liquid comprising desloratadine can further be prepared by dissolving desloratadine in organic solvent(s) or a mixture of water with organic solvent(s). Examples of organic solvents are, but not limited to, lower alcohols such as methanol, ethanol, isopropanol or mixtures thereof.

The at least one stabilizing agent can be added in any step of manufacturing process such as for example dry mixing of excipients, granulation step or in the phase of preparation of compression mixture. Desloratadine can be added in the same phase as stabilizing agent, in particular as one or more of the at least one stabilizing agent(s), or in a separate phase.

The wetting of a mixture of excipient(s) and optionally desloratadine can be performed in conventional granulation equipment by spraying of granulating liquid or granulating liquid comprising desloratadine onto an excipient or mixture of excipients by conventional pharmaceutical techniques. Wetting can also be performed by direct addition of granulating liquid or granulation liquid comprising desloratadine to a mixture of excipients during mixing operation in a proper mixing device, e.g. high-shear mixer.

In the case granulating liquid comprising desloratadine is applied, preferably sprayed, onto an excipient or mixture of excipients amorphous form of active ingredient is formed. As a starting material, e.g. for preparing a granulation liquid or a mixture to be subjected to a compression, desloratadine in amorphous form or different crystalline forms such as polymorphic form I, polymorphic form II, or mixtures thereof, or mixture of amorphous form and any crystalline form can be used.

Mixing of the excipients or of excipients with desloratadine may be performed in conventional devices used for mixing of powders, e.g. motionless (passive) mixers, fluidized bed, diffusion, biconic diffusion, uniconic, biconic, turbular, cubic, planetary, Y-, V-shaped or high-shear mixers.

For drying a granulate, conventional drying devices such as a fluid-bed dryer or drying chambers, e.g. non-vacuumized or vacuumized can be used.

Further, drying a granulate, prepared in a wet granulation process, in a fluid-bed dryer enables the preparation of round-shaped particles of granulate consisting of desloratadine and excipient(s), which provide a reproducible and processible formulation.

In the process according to the present invention as described above, the compression, in particular to cores/tablets, can be performed using an automatic rotary compressing machine from different manufacturers of equipment for use in pharmaceutical industry.

Conventional equipment can be used for applying film coating, such as fluid bed (e.g. Wurster coating system) or conventional coating pans for use in pharmaceutical industry.

It has been found out that the inventive solid pharmaceutical composition prepared by processes according to the present invention is stable at ICH stability testing conditions.

The solid pharmaceutical compositions obtained according to Examples 2 and 3 were compared with a product currently marketed under the name Aerius^{®} (5 mg film coated tablets).

Following an accelerated stability test scheme, compositions were stored at 40 °C/75 % RH for 15 days.

Both before (t = 0) and after exposition (t=15 days) to accelerated stability testing conditions, the amount of total impurities present in the compositions was measured using HPLC (area-%). Moisture content in the compositions was measured by Karl-Fischer method both before (t = 0) and after exposition (t=15 days) to accelerated stability testing conditions. The amount of total impurities and the moisture content is given in the following table:

| Stability testing condition | 40°C and 75% RH, open containers | | | |
|---|---|---|---|---|
| | Total impurities | | Moisture content | |
| | t=0 | t=15 days | t=0 | t=15 days |
| Aerius^{®} 5mg | 0.192 | 0.427 | 4.23 | 4.37 |
| Example 2 | 0.065 | 0.211 | 5.64 | 6.50 |
| Example 3 | 0.063 | 0.191 | 5.55 | 7.10 |

The solid pharmaceutical composition prepared according to the present invention is advantageous over the known prior art and surprisingly shows improved stability when compared with a product currently marketed under the name Aerius^{®} (5 mg film coated tablets). Despite higher moisture uptake of the solid pharmaceutical composition according to the present invention in comparison to a product currently marketed under the name Aerius^{®} (5 mg film coated tablets), unexpectedly and surprisingly, the amount of total impurities is lower, which is contrary to the teaching of the prior art.

The invention also relates to a packaged pharmaceutical composition comprising the solid pharmaceutical composition described above, which is present in a low gas permeable primary packaging.

The low gas permeable primary packaging may comprise materials such as aluminium or polychloro-3-fluoroethylene homopolymer/PVC laminate. Typically, the thickness of the packaging will be in the range of 10 to 40 µm for Al/Al blisters and 10 to 110 µm for Al-polychloro-3-fluoroethylene homopolymer/PVC laminate blisters. Optionally, the packaged pharmaceutical composition may further comprise a desiccant. The desiccant may be placed inside the packaging unit together with a dosage form such as a tablet and/or in the closure system and/or can be incorporated into the walls of the primary packaging unit. For example, the pharmaceutical composition can be packaged in containers made of glass or polymers, with or without desiccant.

The process for the preparation of the solid pharmaceutical composition or the packaged pharmaceutical composition can optionally be performed under conditions of reduced relative humidity of the surrounding atmosphere, e.g. at conditions, wherein the relative humidity of the surrounding atmosphere is below 40 %, preferably below 35 %, to prevent moisture sorption from the surrounding atmosphere into the composition.

To avoid substantial oxidative degradation of active ingredient and other ingredients susceptible to such degradation, the composition may be packaged into a primary packaging, such as a low gas permeable primary packaging, in an inert atmosphere such as nitrogen, argon or xenon. This will provide for a decreased concentration of oxygen in the atmosphere surrounding the dosage form in the primary packaging such as for example a blister, strip, glass or plastic container, such as a securitainer. As used herein, the term "decreased concentration of oxygen" means that the concentration of oxygen in the atmosphere surrounding the individual dosage form such as tablet or capsule is below 10 vol.-%, particularly below 7.5 vol.-%, more preferably below 5 vol.-% and most preferably below 2.5 vol.-%.

The following examples are to further illustrate preferred aspects of the invention without limiting it thereto.

### Examples

### Example 1

| **Dry Mixing** | [mg] |
|---|---|
| Maize Starch | 40.00 |
| Starch, Pregelatinized | 5.00 |
| Lactose monohydrate | 5.00 |
| Cellulose, Microcrystalline | 36.00 |
| **Granulation** | |
| Hypromellose | 3.00 |
| Purified water | Qs |
| **Drug Loading** | |
| Desloratadine | 5.00 |
| Hypromellose | 4.00 |
| Purified water | Qs |
| Conc. HCl | Qs |
| NaOH for 1st spray | 0.008 |
| **Lubrication I** | |
| Purified Talc | 2.00 |
| **Lubrication II** | |
| Cellulose, Microcrystalline | 20.00 |
| **Tablet coating** | |
| Opadry II* | 9.00 |

| | |
|---|---|
| *Composition of Opadry II 85F18422 White: Polyvinyl Alcohol, Polyethylene Glycol, Talc, TiO2 | |

Maize starch, pregelatinised starch, microcrystalline cellulose and lactose monohydrate were homogenised in high-shear mixer.

HPMC was dispersed in purified water to obtain granulating liquid. Granulating liquid was sprayed onto the mixture of excipients as listed above. The granulate obtained was dried in fluid-bed dryer. Loss on drying of the sieved granulate was less than 6.0% by weight.

The dried base granules were loaded in the fluid bed granulator and a solution of NaOH dissolved in water was sprayed onto the granules.

The drug solution was prepared as follows. Concentrated HCl was dissolved in purified water. Desloratadine was added to the solution and stirred until a clear solution was obtained. Finally HPMC was added to the solution to obtain a clear viscous granulating liquid. The above solution was sprayed onto the obtained granules in a fluid bed granulator. The granules were dried and the, loss on drying was below 4.0% by weight.

Finally, talc and microcrystalline cellulose were added to obtain compression mixture.

Cores were coated in an automatic coating pan with water-based film coating suspension of ready-to-make mixture Opadry.

### Example 2

| **Dry Mixing** | [mg] |
|---|---|
| Cellulose, Microcrystalline | 68.00 |
| **Granulation** | |
| NaOH | 0.01 |
| Purified water | qs |
| **Drug Loading** | |
| Desloratadine | 5.00 |
| Hypromellose | 5.00 |
| Conc. HCl | qs |
| Purified water | qs |
| **Lubrication** | |
| Maize starch | 20.00 |
| Purified Talc | 2.00 |
| **Tablet coating** | |
| Opadry II* | 7.50 |

| | |
|---|---|
| * Composition of Opadry II 32F58900 White: Hypromellose, Polyethylene Glycol, Lactose Monohydrate, TiO2 | |

Microcrystalline cellulose was loaded in the fluid bed granulator and a solution of NaOH dissolved in water was sprayed onto the granules.

The drug solution was prepared as follows. Concentrated HCl was dissolved in purified water. Desloratadine was added to the solution and stirred until a clear solution was obtained. Finally HPMC was added to the solution to obtain a clear viscous granulating liquid. The above solution was sprayed onto the mixture in a fluid bed granulator. The granules were dried and the loss on drying was below 3.0% by weight.

Finally, talc and maize starch were added to obtain compression mixture.

Cores were coated in an automatic coating pan with water-based film coating suspension of ready-to-make mixture Opadry.

### Example 3

| **Dry Mixing** | [mg] |
|---|---|
| Cellulose, Microcrystalline | 68.00 |
| **Granulation** | |
| NaOH | 0.01 |
| Purified water | qs |
| **Drug Loading** | |
| Desloratadine | 5.00 |
| Hypromellose | 5.00 |
| Conc. HCl | qs |
| Purified water | qs |
| **Lubrication** | |
| Maize starch | 15.00 |
| Pharmatose DCL 11 | 5.00 |
| Purified Talc | 2.00 |
| **Tablet coating** | |
| Opadry II* | 7.50 |

| | |
|---|---|
| * Composition of Opadry II 32F58900 White: Hypromellose, Polyethylene Glycol, Lactose Monohydrate, TiO2 | |

The drug loaded granules were prepared as described in Example 2. Maize starch, lactose monohydrate and talc were added to obtain the compression mixture.
Cores were coated in an automatic coating pan with water-based film coating suspension of ready-to-make mixture Opadry.

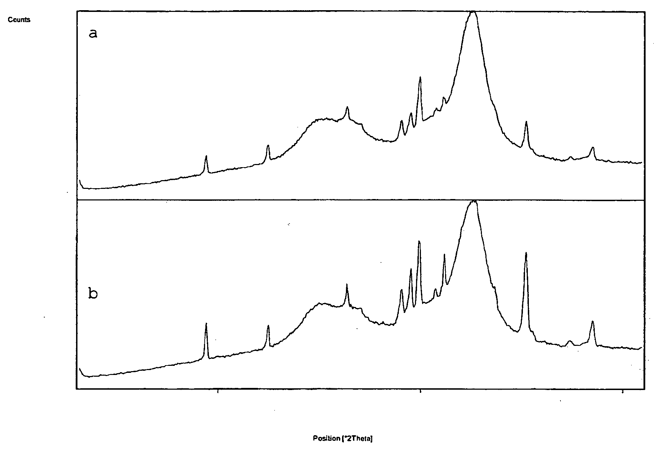

The XRD diffractogram of the solid pharmaceutical composition obtained according to Example 3 (shown in Fig. 1a) is in concordance with the XRD diffractogram of the solid pharmaceutical composition without the active ingredient desloratadine obtained according to Example 3 (shown in Fig. 1b). Desloratadine present in the solid pharmaceutical composition is in the amorphous form.

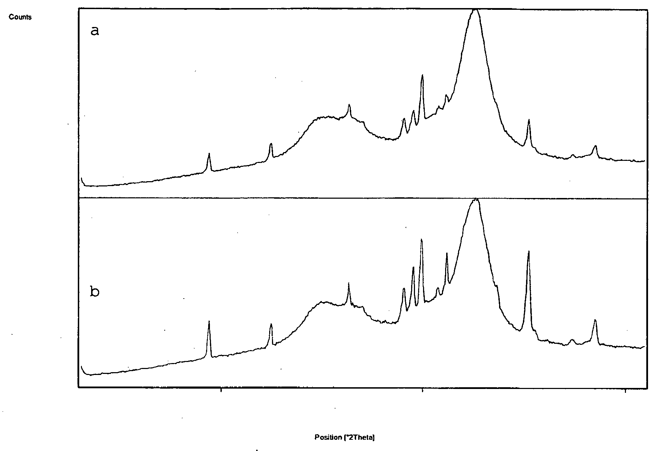

The XRD diffractogram of the solid pharmaceutical composition obtained according to Example 3 measured after 15 days storage in open containers at 40 °C/75 % RH (shown in Fig. 2a) is in concordance with the XRD diffractogram of the solid pharmaceutical composition without the active ingredient desloratadine obtained according to Example 3 measured after 15 days storage in open containers at 40 °C/75 % RH (shown in Fig. 2b). Desloratadine present in the solid pharmaceutical composition is in the amorphous form.

### Example 4

| **Dry Mixing** | [mg] |
|---|---|
| Cellulose, Microcrystalline | 68.00 |
| **Granulation** | |
| NaOH | 0.01 |
| Ethanol | qs |
| **Drug Loading** | |
| Desloratadine | 5.00 |
| Hypromellose | 5.00 |
| Ethanol:Purified water | qs |
| Lubrication | |
| Maize starch | 15.00 |
| Pharmatose DCL 11 | 5.00 |
| Purified Talc | 2.00 |
| **Tablet coating** | |
| Opadry II* | 7.50 |

| | |
|---|---|
| * Composition of Opadry II 32F58900 White: Hypromellose, Polyethylene Glycol, Lactose Monohydrate, TiO2 | |

Microcrystalline cellulose was loaded in the fluid bed granulator and a solution of NaOH dissolved in ethanol was sprayed onto the granules.

The drug solution was prepared as follows. Desloratadine was dissolved in ethanol and stirred until a clear solution was obtained. HPMC was added into the solution and purified water was added to obtain a clear viscous granulating liquid. The above solution was sprayed onto the mixture in a fluid bed granulator. The granules were dried.

Finally, talc, Pharmatose DCL11 and maize starch were added to obtain compression mixture.

Cores were coated in an automatic coating pan with water-based film coating suspension of ready-to-make mixture Opadry.

### Example 5

| **Dry Mixing** | [mg] |
|---|---|
| Cellulose, Microcrystalline | 65.50 |
| **Granulation** | |
| Ca(OH)₂ | 2.50 |
| Purified water | qs |
| **Drug Loading** | |
| Desloratadine | 5.00 |
| Hypromellose | 5.00 |
| Conc. HCl | qs |
| Purified water | qs |
| **Lubrication** | |
| Maize starch | 15.00 |
| Pharmatose DCL 11 | 5.00 |
| Purified Talc | 2.00 |
| **Tablet coating** | |
| Opadry II* | 7.50 |

| | |
|---|---|
| * Composition of Opadry II 32F58900 White: Hypromellose, Polyethylene Glycol, Lactose Monohydrate, TiO2 | |

Microcrystalline cellulose was loaded in the fluid bed granulator and a solution of Ca(OH)₂ dissolved in water was sprayed onto the granules.

The drug solution was prepared as follows. Concentrated HCl was dissolved in purified water. Desloratadine was added to the solution and stirred until a clear solution was obtained. Finally HPMC was added to the solution to obtain a clear viscous granulating liquid. The above solution was sprayed onto the mixture in a fluid bed granulator. The granules were dried.

Maize starch, Pharmatose DCL11 and talc were added to obtain the compression mixture.

Cores were coated in an automatic coating pan with water-based film coating suspension of ready-to-make mixture Opadry.

### Example 6

| **Dry Mixing** | [mg] |
|---|---|
| Cellulose, Microcrystalline | 65.50 |
| **Granulation** | |
| Mg(OH)₂ | 2.50 |
| Purified water | qs |
| **Drug Loading** | |
| Desloratadine | 5.00 |
| Hypromellose | 5.00 |
| Conc. HCl | qs |
| Purified water | qs |
| **Lubrication** | |
| Maize starch | 15.00 |
| Pharmatose DCL 11 | 5.00 |
| Purified Talc | 2.00 |
| **Tablet coating** | |
| Opadry II* | 7.50 |

| | |
|---|---|
| * Composition of Opadry II 32F58900 White: Hypromellose, Polyethylene Glycol, Lactose Monohydrate, TiO2 | |

Microcrystalline cellulose was loaded in the fluid bed granulator and a solution of Mg(OH)₂ dissolved in water was sprayed onto the granules.

The drug solution was prepared as follows. Concentrated HCl was dissolved in purified water. Desloratadin was added to the solution and stirred until a clear solution was obtained. Finally HPMC was added to the solution to obtain a clear viscous granulating liquid. The above solution was sprayed onto the mixture in a fluid bed granulator. The granules were dried.

Maize starch, Pharmatose DCL11 and talc were added to obtain the compression mixture.

Cores were coated in an automatic coating pan with water-based film coating suspension of ready-to-make mixture Opadry.

### Example 7

| **Dry Mixing** | [mg] |
|---|---|
| Desloratadine | 5.00 |
| Celactose 80 | 68.00 |
| Mg(OH)₂ | 10.00 |
| Cellulose, Microcrystalline | 20.00 |
| Croscarmellose sodium | 3.00 |
| Magnesium stearate | 1.00 |
| TOTAL CORES | 107.00 |
| **Tablet coating** | |
| Opadry White* | 3.00 |

| | |
|---|---|
| containing HPMC, titanium dioxide, talc and propylene glycol | |

Desloratadine, Celactose 80, magnesium hydroxide, microcrystalline cellulose and croscarmellose sodium were mixed. Magnesium stearate was admixed and the obtained mixture compressed into cores, which were film-coated using Opadry White. Desloratadine used was in polymorphic form I with average particle size 47 µm.

### Example 8

| **Dry Mixing** | [mg] |
|---|---|
| Cellulose, Microcrystalline | 30.00 |
| Lactose monohydrate | 70.00 |
| **Granulation** | |
| Povidone | 3.00 |
| Sodium hydroxide | 1.00 |
| Purified water | qs |
| **Compression mixture** | |
| Desloratadine | 5.00 |
| Croscarmellose sodium | 3.00 |
| Magnesium stearate | 1.5 |
| **Tablet coating** | |
| Opadry White* | 3.00 |

| | |
|---|---|
| containing HPMC, titanium dioxide, talc and propylene glycol | |

Povidone and sodium hydroxide were dissolved in purified water and sprayed onto the mixture on lactose and microcrystalline cellulose. Obtained granules were dried, sieved and mixed with desloratadine and croscarmellose sodium. Magnesium stearate was admixed and the obtained mixture was compressed into cores, which were further coated with Opadry White. Desloratadine used was in the form of mixture of polymorphic forms I and II (85:15) with average particle size 34 µm.

### Example 9

| **Dry Mixing** | [mg] |
|---|---|
| Cellulose, Microcrystalline | 67.50 |
| **Granulation** | |
| NaOH | 0.50 |
| Purified water | qs |
| **Drug Loading** | |
| Desloratadine | 5.00 |
| Hypromellose | 5.00 |
| Ethanol | qs |
| **Lubrication** | |
| Maize starch | 15.00 |
| Pharmatose DCL 11 | 5.00 |
| Purified Talc | 2.00 |
| **Tablet coating** | |
| Opadry II* | 7.50 |

| | |
|---|---|
| * Composition of Opadry II: Hypromellose, Polyethylene Glycol, Lactose Monohydrate, TiO2 | |

Microcrystalline cellulose was loaded in the fluid bed granulator and a solution of NaOH dissolved in ethanol was sprayed onto the granules.

The drug solution was prepared as follows. Desloratadine was dissolved in ethanol and stirred until a clear solution was obtained. HPMC was added into the solution and stirred to obtain a clear viscous granulating liquid. The above solution was sprayed onto the mixture in a fluid bed granulator. The granules were dried.

Finally, talc, Pharmatose DCL11 and maize starch were added to obtain compression mixture.

Cores were coated in an automatic coating pan with water-based film coating suspension of ready-to-make mixture Opadry.

### Example 10

| **Dry Mixing** | [mg] |
|---|---|
| Cellulose, Microcrystalline | 67.50 |
| **Granulation** | |
| NaOH | 0.50 |
| Purified water | qs |
| **Drug Loading** | |
| Desloratadine | 5.00 |
| Hypromellose | 5.00 |
| Isopropanol | qs |
| **Lubrication** | |
| Maize starch | 15.00 |
| Pharmatose DCL 11 | 5.00 |
| Purified Talc | 2.00 |
| **Tablet coating** | |
| Opadry II* | 7.50 |

| | |
|---|---|
| * Composition of Opadry II: Hypromellose, Polyethylene Glycol, Lactose Monohydrate, TiO2 | |

Microcrystalline cellulose was loaded in the fluid bed granulator and a solution of NaOH dissolved in isopropanol was sprayed onto the granules.
The drug solution was prepared as follows. Desloratadine was dissolved in isopropanol and stirred until a clear solution was obtained. HPMC was added into the solution and stirred to obtain a clear viscous granulating liquid. The above solution was sprayed onto the mixture in a fluid bed granulator. The granules were dried.
Finally, talc, Pharmatose DCL11 and maize starch were added to obtain compression mixture.

Cores were coated in an automatic coating pan with water-based film coating suspension of ready-to-make mixture Opadry.

### Example 11

| **Dry Mixing** | [mg] |
|---|---|
| Cellulose, Microcrystalline | 61.65 |
| **Drug Loading** | |
| Hypromellose | 4.00 |
| Desloratadine | 5.00 |
| Purified water | Qs |
| Conc. HCl | Qs |
| **Granulation** | |
| NaOH | 0.35 |
| Purified water | Qs |
| **Compression mixture** | |
| Pharmatose DCL 11 | 14.00 |
| Maize Starch | 11.00 |
| Talc | 4.00 |
| **Tablet coating** | |
| Opadry II* | 7.50 |
| Opadry Clear** | 1.00 |

| | |
|---|---|
| *Composition of Opadry II: Hypromellose, Polyethylene Glycol, Lactose monohydrate, TiO2. **Composition of Opadry Clear: Hypromellose, Polyethylene Glycol | |

Microcrystalline cellulose was homogenised in Fluid bed granulator
The drug solution was prepared as follows. Concentrated HCl was dissolved in purified water. Desloratadine was added to the solution and stirred until a clear solution was obtained. Finally HPMC was added to the solution to obtain a clear viscous granulating liquid. The above solution was sprayed onto the microcrystalline cellulose in a fluid bed granulator. The granules were dried.
The dried granules were loaded in the fluid bed granulator and a solution of NaOH dissolved in water was sprayed onto the granules.
The granules were dried and the loss on drying was below 4.0% by weight.
Finally, Pharmatose DCL 11, maize starch and talk were added to obtain compression mixture.
Cores were coated in an automatic coating pan with water-based film coating suspension of ready-to-make mixture Opadry II and an additional overcoat of Opadry Clear was applied.

## Claims

1. A solid pharmaceutical composition comprising desloratadine and at least one stabilizing agent selected from the group consisting of alkali metal hydroxides, alkali earth metal hydroxides, and aluminium hydroxide, said solid pharmaceutical composition being prepared by a process for the preparation of a solid pharmaceutical composition, wherein the process comprises or is wet granulation.

2. The solid pharmaceutical composition according to claim 1, wherein said at least one stabilizing agent is selected from the group consisting of NaOH, KOH, Ca(OH)₂, Mg(OH)₂ and Al(OH)₃,
preferably
from the group consisting of NaOH and KOH.

3. The solid pharmaceutical composition according to any of claims 1 to 2, wherein one or more of said stabilizing agent(s) are present in an amount of 0.001 to 10.00 weight%, calculated on the weight of the solid pharmaceutical composition.

4. The solid pharmaceutical composition according to any of claims 1 to 3, wherein desloratadine is partially or completely present in amorphous form,
and wherein optionally
the amorphous form of desloratadine is prepared during the process for the preparation of said solid pharmaceutical composition, said solid pharmaceutical composition being preferably obtainable by a process comprising:
a) providing desloratadine completely or at least partially in a form different from the amorphous form of desloratadine, and
b) converting the desloratadine present in a form different from the amorphous form of desloratadine completely or at least partially into desloratadine in amorphous form.

5. The solid pharmaceutical composition according to any of claims 1 to 3, wherein desloratadine is partially or completely present in crystalline form.

6. The solid pharmaceutical composition according to any of claims 1 to 5, wherein the total weight of stabilizing agents selected from the group consisting of alkali metal hydroxides, alkali earth metal hydroxides, and aluminium hydroxide, preferably selected from the group consisting of NaOH, KOH, Ca(OH)₂, Mg(OH)₂, and Al(OH)₃, more preferably selected from the group consisting of NaOH and KOH, and the total weight of desloratadine present in and/or added during the preparation of the pharmaceutical composition are present in a weight ratio of (total weight of stabilizing agents) : (total weight of desloratadine) of from 0.001 : 1 to 3:1, preferably from 0.01:1 to 2:1, more preferably from 0.1:1 to 1:1,
and/or
wherein desloratadine is present in an amount of 0.1 to 90 weight%, preferably in an amount of 2 to 20 weight%, more preferably in an amount of 3 to 8 weight%, each based on the weight of the solid pharmaceutical composition.

7. A process for the preparation of a solid pharmaceutical composition according to any of claims 1 to 6, wherein the process comprises or is wet granulation.

8. The process for the preparation of a solid pharmaceutical composition according to claim 7, wherein the wet granulation process comprises:
a) granulating a mixture of excipients using a granulation liquid to obtain a granulate,
b) contacting, preferably spraying, the obtained granulate with a dispersion or a solution of a granulation liquid comprising desloratadine,
c) optional addition of further excipient(s) to the granulate, preferably to the granulate resulting from step b), to give a compression mixture,
d) compressing the compression mixture to a desired form, and
e) optionally, applying a coating,
or
the wet granulation process comprises:
a) granulating a mixture of excipient(s), using a granulating liquid comprising desloratadine to obtain a granulate,
b) optional addition of further excipient(s) to the granulate to give a compression mixture,
c) compressing the compression mixture to a desired form, and
d) optionally, applying a coating.

9. The process for the preparation of a solid pharmaceutical composition according to claim 7, wherein the wet granulation process comprises:
a) mixing desloratadine with at least one excipient,
b) granulating the mixture of desloratadine and excipient(s), using granulating liquid to obtain a granulate,
c) optional addition of further excipient(s) to the granulate to give a compression mixture,
d) compression of the obtained mixture to a desired form, and
e) optionally, coating,
or
wherein the wet granulation process comprises:
a) granulating a mixture of excipient(s), using a granulation liquid comprising desloratadine to obtain a granulate,
b) contacting, preferably spraying, the obtained drug loaded granules with granulation liquid,
c) optional addition of further excipient(s) to the granulate to give a compression mixture,
d) compressing the compression mixture to a desired form, and
e) optionally, applying a coating.

10. The process for the preparation of a solid pharmaceutical composition according to claim 7, wherein the wet granulation process comprises:
a) granulating a mixture of excipient(s), using granulation liquid comprising desloratadine to obtain a granulate,
b) contacting, preferably spraying, the obtained drug loaded granules with granulation liquid comprising a stabilizing agent, preferably with a granulation liquid comprising at least one stabilizing agent selected from the group consisting of alkali metal hydroxides, alkali earth metal hydroxides, and aluminium hydroxide,
c) optional addition of further excipient(s) to the granulate to give a compression mixture,
d) compressing the compression mixture to a desired form, and
e) optionally, applying a coating,
or
wherein the wet granulation process comprises:
a) granulating a mixture of excipient(s), using granulation liquid comprising desloratadine to obtain a granulate,
b) contacting, preferably spraying, the obtained drug loaded granules with granulation liquid comprising stabilizing agent(s) selected from sodium hydroxide and potassium hydroxide,
c) optional addition of further excipient(s) to the granulate, preferably to the granulate resulting from step b), to give a compression mixture,
d) compressing the compression mixture to a desired form, and
e) optionally, applying a coating.

11. The process for the preparation of a solid pharmaceutical composition according to any of claims 7 to 10, which further comprises
a) providing desloratadine completely or at least partially present in a form different from the amorphous form of desloratadine,
b) preparing a granulation liquid from said desloratadine completely or at least partially present in a form different from the amorphous form of desloratadine.

12. The process for the preparation of a solid pharmaceutical composition according to any of claims 7 to 11, which further comprises
- obtaining a pharmaceutical composition comprising desloratadine completely or at least partially present in amorphous form, preferably obtaining a pharmaceutical composition having a weight ratio of desloratadine present in amorphous form to desloratadine present in a form different from amorphous form which is higher than the weight ratio of desloratadine present in amorphous form to desloratadine present in a form different from amorphous form present in the desloratadine provided for preparing the granulation liquid comprising desloratadine.

13. Use of a pharmaceutical composition according to any of the claims 1 to 6 for the manufacture of a medicament for treating, preventing or alleviating allergic rhinitis and/or urticaria, in particular sneezing and itching associated with allergic rhinitis, and nasal congestion.

14. The pharmaceutical composition according to any of the claims 1 to 6 for use in treatment, prevention or alleviation of allergic rhinitis and/or urticaria, in particular sneezing and itching associated with allergic rhinitis, and nasal congestion.

## Patentansprüche

1. Feste pharmazeutische Zusammensetzung, umfassend Desloratadin und mindestens ein stabilisierendes Mittel, das aus der Gruppe ausgewählt ist, die aus Alkalimetallhydroxiden, Erdalkalimetallhydroxiden und Aluminiumhydroxid besteht, wobei die feste pharmazeutische Zusammensetzung durch ein Verfahren zur Herstellung einer festen pharmazeutischen Zusammensetzung hergestellt wird, wobei das Verfahren Feuchtgranulierung umfasst oder ist.

2. Feste pharmazeutische Zusammensetzung gemäß Anspruch 1, wobei das mindestens eine stabilisierende Mittel aus der Gruppe, die aus NaOH, KOH, Ca(OH)₂, Mg(OH)₂ und Al(OH)₃ besteht,
bevorzugt aus der Gruppe, die aus NaOH und KOH besteht, ausgewählt ist.

3. Feste pharmazeutische Zusammensetzung gemäß einem der Ansprüche 1 bis 2, wobei eines oder mehrere von de(n/m) stabilisierenden Mittel(n) in einer Menge von 0,001 bis 10,00 Gew.-%, berechnet auf das Gewicht der festen pharmazeutischen Zusammensetzung, vorhanden ist/sind.

4. Feste pharmazeutische Zusammensetzung gemäß einem der Ansprüche 1 bis 3, wobei Desloratadin teilweise oder vollständig in amorpher Form vorhanden ist,
und wobei gegebenenfalls die amorphe Form von Desloratadin während dem Verfahren zur Herstellung der festen pharmazeutischen Zusammensetzung hergestellt wird, wobei die feste pharmazeutische Zusammensetzung bevorzugt erhalten werden kann durch ein Verfahren, umfassend:
a) Bereitstellen von Desloratadin vollständig oder mindestens teilweise in einer Form, die unterschiedlich von der amorphen Form von Desloratadin ist, und
b) Umwandeln des Desloratadins, das in einer Form vorhanden ist, die unterschiedlich von der amorphen Form von Desloratadin ist, vollständig oder mindestens teilweise in Desloratadin in amorpher Form.

5. Feste pharmazeutische Zusammensetzung gemäß einem der Ansprüche 1 bis 3, wobei Desloratadin teilweise oder vollständig in kristalliner Form vorhanden ist.

6. Feste pharmazeutische Zusammensetzung gemäß einem der Ansprüche 1 bis 5, wobei das Gesamtgewicht an stabilisierenden Mitteln, die aus der Gruppe ausgewählt sind, welche aus Alkalimetallhydroxiden, Erdalkalimetallhydroxiden und Aluminiumhydroxid besteht, die bevorzugt aus der Gruppe ausgewählt sind, welche aus NaOH, KOH, Ca(OH)₂, Mg(OH)₂ und Al(OH)₃ besteht, die stärker bevorzugt aus der Gruppe ausgewählt sind, welche aus NaOH und KOH besteht, und das Gesamtgewicht an Desloratadin, das in der pharmazeutischen Zusammensetzung vorhanden ist und/oder während der Herstellung davon zugegeben wird, in einem Gewichtsverhältnis von (Gesamtgewicht an stabilisierenden Mitteln) : (Gesamtgewicht an Desloratadin) von 0,001:1 bis 3:1, bevorzugt 0,01:1 bis 2:1, stärker bevorzugt 0,1:1 bis 1:1 vorhanden sind,
und/oder wobei Desloratadin in einer Menge von 0,1 bis 90 Gew.-%, bevorzugt in einer Menge von 2 bis 20 Gew.-%, stärker bevorzugt in einer Menge von 3 bis 8 Gew.-%, jeweils bezogen auf das Gewicht der festen pharmazeutischen Zusammensetzung, vorhanden ist.

7. Verfahren zur Herstellung einer festen pharmazeutischen Zusammensetzung gemäß einem der Ansprüche 1 bis 6, wobei das Verfahren Feuchtgranulierung umfasst oder ist.

8. Verfahren zur Herstellung einer festen pharmazeutischen Zusammensetzung gemäß Anspruch 7, wobei das Feuchtgranulierungsverfahren umfasst:
a) Granulieren eines Gemisches von Exzipienten unter Verwendung einer Granulierflüssigkeit, um ein Granulat zu erhalten,
b) Inkontaktbringen, bevorzugt Besprühen, des erhaltenen Granulats mit einer Dispersion oder einer Lösung einer Granulierflüssigkeit, umfassend Desloratadin,
c) gegebenenfalls Geben von einem oder mehreren weiteren Exzipienten zu dem Granulat, bevorzugt zu dem Granulat, das aus Schritt b) resultiert, um ein Kompressionsgemisch zu ergeben,
d) Komprimieren des Kompressionsgemisches zu einer gewünschten Form, und
e) gegebenenfalls Aufbringen eines Überzugs,
oder das Feuchtgranulierungsverfahren umfasst:
a) Granulieren eines Gemisches von Exzipient(en) unter Verwendung einer Granulierflüssigkeit, umfassend Desloratadin, um ein Granulat zu erhalten,
b) gegebenenfalls Geben von einem oder mehreren weiteren Exzipienten zu dem Granulat, um ein Kompressionsgemisch zu ergeben,
c) Komprimieren des Kompressionsgemisches zu einer gewünschten Form, und
d) gegebenenfalls Aufbringen eines Überzugs.

9. Verfahren zur Herstellung einer festen pharmazeutischen Zusammensetzung gemäß Anspruch 7, wobei das Feuchtgranulierungsverfahren umfasst:
a) Mischen von Desloratadin mit mindestens einem Exzipienten,
b) Granulieren des Gemisches von Desloratadin und Exzipient(en) unter Verwendung einer Granulierflüssigkeit, um ein Granulat zu erhalten,
c) gegebenenfalls Geben von einem oder mehreren weiteren Exzipienten zu dem Granulat, um ein Kompressionsgemisch zu ergeben,
d) Komprimieren des erhaltenen Gemisches zu einer gewünschten Form, und
e) gegebenenfalls Überziehen,
oder wobei das Feuchtgranulierungsverfahren umfasst:
a) Granulieren eines Gemisches von Exzipient(en) unter Verwendung einer Granulierflüssigkeit, umfassend Desloratadin, um ein Granulat zu erhalten,
b) Inkontaktbringen, bevorzugt Besprühen, der erhaltenen mit Arzneistoff beladenen Granula mit einer Granulierflüssigkeit,
c) gegebenenfalls Geben von einem oder mehreren weiteren Exzipienten zu dem Granulat, um ein Kompressionsgemisch zu ergeben,
d) Komprimieren des Kompressionsgemisches zu einer gewünschten Form, und
e) gegebenenfalls Aufbringen eines Überzugs.

10. Verfahren zur Herstellung einer festen pharmazeutischen Zusammensetzung gemäß Anspruch 7, wobei das Feuchtgranulierungsverfahren umfasst:
a) Granulieren eines Gemisches von Exzipient(en) unter Verwendung einer Granulierflüssigkeit, umfassend Desloratadin, um ein Granulat zu erhalten,
b) Inkontaktbringen, bevorzugt Besprühen, der erhaltenen mit Arzneistoff beladenen Granula mit einer Granulierflüssigkeit, umfassend ein stabilisierendes Mittel, bevorzugt mit einer Granulierflüssigkeit, umfassend mindestens ein
stabilisierendes Mittel, das aus der Gruppe ausgewählt ist, welche aus Alkalimetallhydroxiden, Erdalkalimetallhydroxiden und Aluminiumhydroxid besteht,
c) gegebenenfalls Geben von einem oder mehreren weiteren Exzipienten zu dem Granulat, um ein Kompressionsgemisch zu ergeben,
d) Komprimieren des Kompressionsgemisches zu einer gewünschten Form, und
e) gegebenenfalls Aufbringen eines Überzugs,
oder wobei das Feuchtgranulierungsverfahren umfasst:
a) Granulieren eines Gemisches von Exzipient(en) unter Verwendung einer Granulierflüssigkeit, umfassend Desloratadin, um ein Granulat zu erhalten,
b) Inkontaktbringen, bevorzugt Besprühen, der erhaltenen mit Arzneistoff beladenen Granula mit einer Granulierflüssigkeit, umfassend (ein) stabilisierende(s) Mittel, ausgewählt aus Natriumhydroxid und Kaliumhydroxid,
c) gegebenenfalls Geben von einem oder mehreren weiteren Exzipienten zu dem Granulat, bevorzugt zu dem Granulat, das aus Schritt b) resultiert, um ein Kompressionsgemisch zu ergeben,
d) Komprimieren des Kompressionsgemisches zu einer gewünschten Form, und
e) gegebenenfalls Aufbringen eines Überzugs.

11. Verfahren zur Herstellung einer festen pharmazeutischen Zusammensetzung gemäß einem der Ansprüche 7 bis 10, welches ferner umfasst
a) Bereitstellen von Desloratadin, das vollständig oder mindestens teilweise in einer Form vorhanden ist, die unterschiedlich von der amorphen Form von Desloratadin ist,
b) Herstellen einer Granulierflüssigkeit von dem Desloratadin, das vollständig oder mindestens teilweise in einer Form vorhanden ist, die unterschiedlich von der amorphen Form von Desloratadin ist.

12. Verfahren zur Herstellung einer festen pharmazeutischen Zusammensetzung gemäß einem der Ansprüche 7 bis 11, welches ferner umfasst
- Erhalten einer pharmazeutischen Zusammensetzung, umfassend Desloratadin, das vollständig oder mindestens teilweise in amorpher Form vorhanden ist, bevorzugt Erhalten einer pharmazeutischen Zusammensetzung mit einem Gewichtsverhältnis
von Desloratadin, das in amorpher Form vorhanden ist, zu Desloratadin, das in einer Form vorhanden ist, die unterschiedlich von einer amorphen Form ist, das höher ist als das Gewichtsverhältnis von Desloratadin, das in amorpher Form vorhanden ist, zu Desloratadin, das in einer Form vorhanden ist, die unterschiedlich von einer amorphen Form ist, die in dem Desloratadin vorhanden ist, das zur Herstellung der Granulierflüssigkeit, umfassend Desloratadin, bereitgestellt wird.

13. Verwendung einer pharmazeutischen Zusammensetzung gemäß einem der Ansprüche 1 bis 6 zur Herstellung eines Medikaments zur Behandlung, Vorbeugung oder Linderung von allergischer Rhinitis und/oder Urtikaria, insbesondere Niesen und Jucken in Zusammenhang mit allergischer Rhinitis, und verstopfter Nase.

14. Pharmazeutische Zusammensetzung gemäß einem der Ansprüche 1 bis 6 zur Verwendung in der Behandlung, Vorbeugung oder Linderung von allergischer Rhinitis und/oder Urtikaria, insbesondere Niesen und Jucken in Zusammenhang mit allergischer Rhinitis, und verstopfter Nase.

## Revendications

1. Composition pharmaceutique solide comprenant la desloratadine et au moins un agent stabilisant choisi parmi le groupe consistant en des hydroxydes de métal alcalin, des hydroxydes de métal alcalino-terreux, et l'hydroxyde d'aluminium, ladite composition pharmaceutique solide étant préparée par un procédé de préparation d'une composition pharmaceutique solide, où le procédé comprend ou est la granulation au mouillé.

2. Composition pharmaceutique solide selon la revendication 1, où ledit au moins un agent stabilisant est choisi parmi le groupe consistant en NaOH, KOH, Ca(OH)₂, Mg(OH)₂ et Al(OH)₃,
de préférence parmi le groupe consistant en NaOH et KOH.

3. Composition pharmaceutique solide selon l'une quelconque des revendications 1 à 2, où un ou plusieurs desdits agents stabilisants sont présents en une quantité allant de 0,001 à 10,00% en poids, calculé sur le poids de la composition pharmaceutique solide.

4. Composition pharmaceutique solide selon l'une quelconque des revendications 1 à 3, où la desloratadine est partiellement ou complètement présente sous forme amorphe,
et où le cas échéant, la forme amorphe de la desloratadine est préparée pendant le procédé de préparation de ladite composition pharmaceutique solide, ladite composition pharmaceutique solide pouvant être obtenue de préférence, par un procédé comprenant :
a) l'alimentation de la desloratadine, complètement ou au moins partiellement sous une forme différente de la forme amorphe de la desloratadine, et
b) la conversion de la desloratadine présente sous une forme différente de la forme amorphe de la desloratadine, complètement ou au moins partiellement en desloratadine de forme amorphe.

5. Composition pharmaceutique solide selon l'une quelconque des revendications 1 à 3, où la desloratadine est partiellement ou complètement présente sous forme cristalline.

6. Composition pharmaceutique solide selon l'une quelconque des revendications 1 à 5, où le poids total des agents stabilisants choisis parmi le groupe consistant en des hydroxydes de métal alcalin, des hydroxydes de métal alcalino-terreux, et l'hydroxyde d'aluminium, de préférence choisi parmi le groupe consistant en NaOH, KOH, Ca(OH)₂, Mg(OH)₂ et Al(OH)₃, de manière plus préférée parmi le groupe consistant en NaOH et KOH, et le poids total de la desloratadine présente dans et/ou ajoutée pendant la préparation de la composition pharmaceutique sont présents en un rapport pondéral de (poids total des agents stabilisants):(poids total de la desloratadine) allant de 0,001:1 à 3:1, de préférence de 0,01:1 à 2:1, de manière plus préférée de 0,1:1 à 1:1,
et/ou
où la desloratadine est présente en une quantité allant de 0,1 à 90% en poids, de préférence en une quantité allant de 2 à 20% en poids, de manière plus préférée en une quantité allant de 3 à 8% en poids, chaque fois sur base du poids de la composition pharmaceutique solide.

7. Procédé de préparation d'une composition pharmaceutique solide selon l'une quelconque des revendications 1 à 6, où le procédé comprend ou est la granulation au mouillé.

8. Procédé de préparation d'une composition pharmaceutique solide selon la revendication 7, où le procédé de granulation au mouillé comprend :
a) la granulation d'un mélange d'excipients en utilisant un liquide de granulation pour obtenir un granulé,
b) la mise en contact, de préférence par pulvérisation, du granulé obtenu avec une dispersion ou une solution d'un liquide de granulation comprenant la desloratadine,
c) l'addition facultative d'autre(s) excipient(s) au granulé, de préférence au granulé résultant de l'étape b), pour donner un mélange de compression,
d) la compression du mélange de compression en une forme souhaitée, et
e) le cas échéant, l'application d'un enrobage,
ou
le procédé de granulation au mouillé comprend :
a) la granulation d'un mélange d'excipient(s) en utilisant un liquide de granulation comprenant la desloratadine pour obtenir un granulé,
b) l'addition facultative d'autre(s) excipient(s) au granulé pour donner un mélange de compression,
c) la compression du mélange de compression en une forme souhaitée, et
d) le cas échéant, l'application d'un enrobage.

9. Procédé de préparation d'une composition pharmaceutique solide selon la revendication 7, où le procédé de granulation au mouillé comprend :
a) le mélange de la desloratadine avec au moins un excipient,
b) la granulation du mélange de desloratadine et d'excipient(s) en utilisant un liquide de granulation pour obtenir un granulé,
c) l'addition facultative d'autre(s) excipient(s) au granulé pour donner un mélange de compression,
d) la compression du mélange de compression en une forme souhaitée, et
e) le cas échéant, l'application d'un enrobage,
ou
où le procédé de granulation au mouillé comprend :
a) la granulation d'un mélange d'excipient(s) en utilisant un liquide de granulation comprenant la desloratadine pour obtenir un granulé,
b) la mise en contact, de préférence par pulvérisation, du granulé chargé de médicament obtenu avec un liquide de granulation,
c) l'addition facultative d'autre(s) excipient(s) au granulé pour donner un mélange de compression,
d) la compression du mélange de compression en une forme souhaitée, et
e) le cas échéant, l'application d'un enrobage.

10. Procédé de préparation d'une composition pharmaceutique solide selon la revendication 7, où le procédé de granulation au mouillé comprend :
a) la granulation d'un mélange d'excipient(s) en utilisant un liquide de granulation comprenant la desloratadine pour obtenir un granulé,
b) la mise en contact, de préférence par pulvérisation, du granulé chargé de médicament obtenu avec un liquide de granulation comprenant un agent de stabilisation, de préférence avec un liquide de granulation comprenant au moins un agent stabilisant choisi parmi le groupe consistant en des hydroxydes de métal alcalin, des hydroxydes de métal alcalino-terreux, et l'hydroxyde d'aluminium,
c) l'addition facultative d'autre(s) excipient(s) au granulé pour donner un mélange de compression,
d) la compression du mélange de compression en une forme souhaitée, et
e) le cas échéant, l'application d'un enrobage,
ou
où le procédé de granulation au mouillé comprend :
a) la granulation d'un mélange d'excipient(s) en utilisant un liquide de granulation comprenant la desloratadine pour obtenir un granulé,
b) la mise en contact, de préférence par pulvérisation, du granulé chargé de médicament obtenu avec un liquide de granulation comprenant un/des agent(s) de stabilisation choisi(s) parmi l'hydroxyde de sodium et l'hydroxyde de potassium,
c) l'addition facultative d'autre(s) excipient(s) au granulé, de préférence au granulé résultant de l'étape b), pour donner un mélange de compression,
d) la compression du mélange de compression en une forme souhaitée, et
e) le cas échéant, l'application d'un enrobage.

11. Procédé de préparation d'une composition pharmaceutique solide selon l'une quelconque des revendications 7 à 10, qui comprend en outre :
a) l'alimentation de la desloratadine, complètement ou au moins partiellement présente sous une forme différente de la forme amorphe de la desloratadine,
b) la préparation d'un liquide de granulation à partir de la desloratadine complètement ou au moins partiellement présente sous une forme différente de la forme amorphe de la desloratadine.

12. Procédé de préparation d'une composition pharmaceutique solide selon l'une quelconque des revendications 7 à 11, qui comprend en outre :
- l'obtention d'une composition pharmaceutique comprenant la desloratadine complètement ou au moins partiellement sous forme amorphe, de préférence l'obtention d'une composition pharmaceutique ayant un rapport pondéral de la desloratadine présente sous forme amorphe à la desloratadine présente sous une forme différente de la forme amorphe, qui est supérieur au rapport pondéral de la desloratadine présente sous forme amorphe à la desloratadine présente sous une forme différente de la forme amorphe présente dans la desloratadine alimentée pour préparer le liquide de granulation comprenant la desloratadine.

13. Utilisation d'une composition pharmaceutique selon l'une quelconque des revendications 1 à 6, pour la préparation d'un médicament pour le traitement, la prévention ou le soulagement de la rhinite allergique et/ou de l'urticaire, en particulier les éternuements et les démangeaisons associés à la rhinite allergique, et la congestion nasale.

14. Composition pharmaceutique selon l'une quelconque des revendications 1 à 6, à utiliser dans le traitement, la prévention ou le soulagement de la rhinite allergique et/ou de l'urticaire, en particulier les éternuements et les démangeaisons associés à la rhinite allergique, et la congestion nasale.
